# EUROPEAN PATENT SPECIFICATION

(11) **EP 1 736 094 B1**
(45) Date of publication and mention of the grant of the patent: **11.01.2017**
(21) Application number: 05728550.4
(22) Date of filing: 11.04.2005
(51) Int. Cl.: A61B 1/00, A61B 1/045, A61B 1/06, A61B 1/005

(54) **ENDOSCOPE DEVICE**
ENDOSKOPVORRICHTUNG
DISPOSITIF D'ENDOSCOPE

(30) Priority: 12.04.2004 JP 2004116683
(43) Date of publication of application: 27.12.2006
(73) Proprietor: Olympus Corporation, Tokyo 192-8507 (JP)
(72) Inventor: MIYAMOTO, Shinichi, Hachioji-shi, Tokyo 192-0916 (JP); KINOUCHI, Hideaki, Hachioji-shi, Tokyo 192-0032 (JP); NAKAMURA, Takeaki, Hino-shi, Tokyo191-0065 (JP); WATANABE, Katsushi, Hachioji-shi, Tokyo 193-0824 (JP)
(74) Representative: von Hellfeld, Axel
(86) International application number: PCT/JP2005/007011
(87) International publication number: WO 2005/099560

(56) References cited:
- EP-A2- 1 300 599
- WO-A1-96/39918
- JP-A- 1 302 216
- JP-A- 1 302 216
- JP-A- 11 009 548
- JP-A- 2000 116 599
- JP-A- 2001 112 704
- US-A1- 2002 161 280
- US-B1- 6 636 254
- US-B1- 6 636 254

## Description

### TECHNICAL FIELD

The present invention relates to an endoscope device that is integrated with an image display device and is suitable for carrying.

### BACGROUND ART OF THE INVENTION

Among endoscope devices that are widely used in medical fields and industrial fields, there are those in which a fixed type of television monitor is left permanently connected via a cable to a portion that corresponds to an eyepiece portion, and an image obtained by the endoscope is formed on a light receiving portion of an imaging element such as a CCD. This formed image is then converted into a signal and the image that has been converted into a signal is supplied via a cable to a television monitor in a separate location. The signal is then converted into an image and displayed on the screen of this television monitor.

Endoscope devices have also been proposed (for example, refer to PATENT DOCUMENTS 1 to 3) in which a light source apparatus, an image display device such as a miniature liquid crystal monitor, and a battery that drives the light source, imaging elements, and the liquid crystal monitor are incorporated in an endoscope. In the above conventional endoscope devices, the image display device is mounted at a top portion of the endoscope, and an image of a subject is displayed on the screen thereof. An image processing display device has also been proposed (refer to PATENT DOCUMENT 4) that rotates an image displayed on a screen relative to a frame of the screen.
PATENT DOCUMENT 1: Japanese Patent Application, First Publication, No. H10-127575
PATENT DOCUMENT 2: Japanese Patent Application, First Publication, No. H11-954
PATENT DOCUMENT 3: Japanese Patent Application, First Publication, No. 2000-116599
PATENT DOCUMENT 4: United States Patent No. 6,636,254

JP 1 302216 A discloses an at-hand operating part of an endoscope that is provided with a TV monitor, wherein its monitor main body can rotate 180 degrees and a panel board having control command means for adjusting a display image and buttons for adjusting density, tone, and brightness. When the use of the endoscope requires its turn, the monitor can be turned to the direction in which inspection is carried out.

US 6 636 254 B1 concerns an endoscope system, in which a surgeon A holds an endoscope with a TV camera (hereinafter, an endoscope) and a therapeutic appliance such as forceps and carries out a surgical procedure while viewing a first monitor. A surgeon B holds a therapeutic appliance such as forceps and carries out the surgical procedure while viewing a second monitor. A video signal sent from the TV camera of the endoscope is fed to and processed by an image processing apparatus, and displayed on each of the first and second monitors. The image processing apparatus transfers the video signal sent from the endoscope to each of an image inverting circuit and a selector switch. The image inverting circuit inverts an image (laterally (to produce a mirror image), vertically, or vertically and laterally (180 DEG )), and supplies a processed video signal to the selector switch. The selector switch selects a video signal to be supplied to each of the first and second monitors, which are display units, in response to a control signal sent from a selector.

### DETAILED DESCRIPTION OF THE INVENTION

The invention is as defined in the appended claims.

### PROBLEMS TO BE SOLVED BY THE INVENTION

When an operator performs an operation or an examination using an endoscope device, the operator may suitably alter the orientation and angle of the video image displaying device in order to make the video image displaying device easier to see. For example, if the video image displaying device is rotated by an arbitrary angle around an axis that is substantially orthogonal to the screen, then the orientation of the screen of the video image displaying device does not change greatly, however, the screen itself rotates around an axis that is substantially orthogonal to the screen. If this type of operation is performed, the video images displayed on the screen become tilted as viewed by the operator and any accompanying assistants, and there is a change in how the video images appear from how they appeared before this operation was performed. As a result, there is a possibility that difficulties will arise as an operation or examination progresses.

The present invention was conceived in view of the above described circumstances and it is an object thereof to provide an endoscope device that makes it possible for an operator and accompanying assistants to obtain an excellent view of an image of a subject that is displayed on a screen.

### MEANS FOR SOLVING THE PROBLEM

Some or all of the above problems are overcome by an endoscope device according to claim 1.

The endoscope device of the present invention is provided with an imaging device for imaging an observation image of a subject, an endoscope having a grippable operation section, a video image displaying device for converting an image signal from the imaging device into a video image and displaying it, a first supporting section that is provided in the operation section and supports the video image displaying device so that the video image displaying device is pivotable around a longitudinal axis of the operation section or about an axis substantially parallel to the longitudinal axis, and a video image rotating device for rotating the image of the subject displayed on the video image displaying device about an axis substantially orthogonal to the screen of the video image displaying device.

In the endoscope device of the present invention, using a video image rotating device it is possible to rotate an image of a subject that is displayed on the screen of a video image displaying device around an axis that is substantially orthogonal to the screen. Therefore, if the screen itself is rotated around an axis that is substantially orthogonal to the screen (i.e., substantially equivalent to the longitudinal axis of the operation section or an axis that is substantially parallel with the longitudinal axis) as a result of the operator altering the orientation and angle of the video image display device, the video image that is displayed on the screen of the video image displaying device is rotated in the opposite direction by an arbitrary angle amount. By employing this type of structure, even if the screen itself is tilted, the way in which a video image appears as seen by the operator is the same as it was before the angle of the video image displaying device was altered. Accordingly, difficulties do not arise as an operation or examination progresses.

In the endoscope device of the present invention, it is preferable for there to be further provided a direction and angle detecting device that, when the video image displaying device is pivoted relative to the operation section, detects a direction in which the video image displaying device has been pivoted and an angle by which it has been pivoted, and for the video image rotating device to rotate the image of the subject that is displayed on the video image displaying device in an opposite direction from the pivot direction detected by the direction and angle detecting device and by the same amount as the pivot angle detected by the direction and angle detecting device.

In the endoscope device of the present invention, when an operator alters the way in which they are holding the endoscope, the direction in which the video image display device has been pivoted and the angle by which it has been pivoted relative to the operation section are detected. In addition, an image of the subject that is displayed on the video image displaying device is rotated in the opposite direction from the pivot direction detected by the direction and angle detecting device and by the same amount as the pivot angle detected by the direction and angle detecting device. As a result, even if the screen itself is tilted, the way in which the video images appear is the same as it was before the angle of the video image display device was altered.

In the endoscope device of the present invention it is preferable for there to be further provided a second supporting section that supports the video image displaying device so that the video image displaying device is pivotable around an axis that is perpendicular to the longitudinal axis or to an axis that is substantially parallel with the longitudinal axis.

In the endoscope device of the present invention, when an operator changes the way they are holding an endoscope and thereby changes the orientation of the video image displaying device so that the line of vision of the operator no longer matches the viewing angle of the screen, by adjusting the video image displaying device by rotating it around an axis that is perpendicular to the longitudinal axis or perpendicular to an axis that is substantially parallel with the longitudinal axis so that the line of vision of the operator enters the viewing angle of the screen, the video image displayed on the screen can be viewed easily in any situation.

In the endoscope device of the present invention, it is preferable for the first and second supporting sections to be able to arbitrarily fix the position of the video image displaying device relative to the operation section and maintain the relative positions of the two as long as there is no intentional external application of pressure.

In the endoscope device of the present invention, the relative positions of the operation section and the video image displaying device are maintained by the first and second supporting sections as long as there is no intentional external application of pressure. Namely, even if force that has not been intentionally applied to the video image displaying device by an operator (for example, gravity or inertial force that is generated when the endoscope device is abruptly moved) is applied thereto, the relative positions of the operation section and the video image displaying device are kept unchanged. Consequently, the endoscope device is easy to handle.

In the endoscope device of the present invention, it is preferable for there to be further provided a pivot drive device that drives the video image displaying device in a pivot direction around the longitudinal axis or around an axis that substantially parallel with the longitudinal axis.

In the endoscope device of the present invention, because it is possible to drive the video image displaying device in a pivot direction around the longitudinal axis or around an axis that is substantially parallel with the longitudinal axis, the operability of the endoscope device is improved.

In the endoscope device of the present invention, it is preferable for there to be provided a rotation operation section that is operated by the operator or by another person, and for the video image rotating device to rotate an image of the subject displayed on the video image displaying device in a predetermined direction in accordance with an operation of the rotation operation section.

In the endoscope device of the present invention, if the rotation operation section is operated, an image of a subject that is displayed on the video image displaying device can be rotated in a predetermined direction. As a result, an operator or another person such as an assistant is able to arbitrarily rotate the image of the subject displayed on the video image displaying device so that they can easily view it.

In the endoscope device of the present invention, it is preferable for there to be further provided an image recording device that records an image of the subject displayed on the video image displaying device, and an image recording selection device that selects whether to record on the image recording device an image of the subject that is displayed on the video image displaying device in a state prior to the video image rotating device being operated or in a state after the video image rotating device has been operated.

In the endoscope device of the present invention, it is possible for an operator or another person such as an assistant to arbitrarily select whether an image to be recorded on the image recording device is to be recorded in a state prior to the video image rotation device being operated or is to be recorded in a state after the video image rotation device has been operated.

### ADVANTAGEOUS EFFECTS OF THE INVENTION

According to the endoscope device of the present invention, by altering the angle of the video image displaying device, even if the screen itself is tilted, how a video image that is displayed on the screen appears as seen by an operator is the same as it was before the angle of the video image displaying device was altered. Accordingly, it is possible to obtain an excellent view of an image of a subject that is displayed on a screen.

### BRIEF DESCRIPTION OF THE DRAWINGS

[FIG. 1] FIG. 1 is a view showing a first embodiment of the endoscope device of the present invention and is a perspective view of the endoscope device as seen from a diagonally upward direction.
[FIG. 2] FIG. 2 is a perspective view seen from a different direction that that in FIG. 1 of the endoscope device of the first embodiment.
[FIG. 3] FIG. 3 is a schematic view showing an internal structure of the endoscope device of the first embodiment.
[FIG. 4] FIG. 4 is a functional block diagram of the endoscope device of the first embodiment.
[FIG. 5] FIG. 5 is a plan view showing an image of a subject that is displayed on the video image displaying device of the first embodiment.
[FIG. 6] FIG. 6 is a plan view showing an image of a subject that is displayed on the video image displaying device of the first embodiment.
[FIG. 7] FIG. 7 is a flowchart of processing to rotate an image of a subject that is displayed on the video image displaying device of the first embodiment.
[FIG. 8] FIG. 8 is a view showing a variant example of the first embodiment and is a schematic view showing the internal structure of an endoscope device.
[FIG. 9] FIG. 9 is a view showing another variant example of the first embodiment and is a schematic view showing the structure of a portion that supports the video image displaying device.
[FIG. 10] FIG. 9 is a view showing a second embodiment of the endoscope device of the present invention and is an exploded perspective view showing principal portions of the endoscope device.
[FIG. 11] FIG. 10 is a schematic view showing an internal structure of the endoscope device of the second embodiment.
[FIG. 12] FIG. 12 is a view showing a third embodiment of the endoscope device of the present invention and is a perspective view of the endoscope device as seen from a diagonally upward direction.
[FIG. 13] FIG. 13 is a schematic view showing an internal structure of the endoscope device of the third embodiment.
[FIG. 14] FIG. 14 is a flowchart of processing to rotate an image of a subject that is displayed on the video image displaying device of the third embodiment.
[FIG. 15] FIG. 15 is a view showing a fourth embodiment of the endoscope device of the present invention and is a perspective view of the endoscope device as seen from a diagonally upward direction.
[FIG. 16] FIG. 16 is a functional block diagram of the endoscope device of the fourth embodiment.
[FIG. 17] FIG. 17 is a plan view showing an initial setting screen that is displayed on the video image displaying device of the fourth embodiment.
[FIG. 18] FIG. 18 is a perspective view showing principal portions of an endoscope device to which the present invention can be applied in addition to the above described respective embodiments of the present invention.
[FIG. 19] FIG. 19 is also a perspective view showing principal portions of an endoscope device to which the present invention can be applied in addition to the above described respective embodiments of the present invention.

### DESCRIPTION OF THE REFERENCE SYMBOLS

1··· Endoscope, 3, 60, 0, 80 ··· Video image displaying device, 4 ··· Imaging element (imaging device), 12 ··· Operation section, 32 ··· Supporting portion (first supporting portion), 38 ··· Display element control circuit (video image display control device), 43 ··· Slip ring, 44 ··· Encoder (direction and angle detecting device), 45 ··· Hinge portion (second supporting portion), 53 ··· Pivot slide mechanism, 42 ··· Motor (pivot drive device), 72 ··· Right rotation button (rotation operation portion), 73 ··· Left rotation button (rotation operation portion, 84 ··· Image recording selection circuit (image recording selection device)

### BEST MOODE FOR CARRYING OUT THE INVENTION

### (First embodiment)

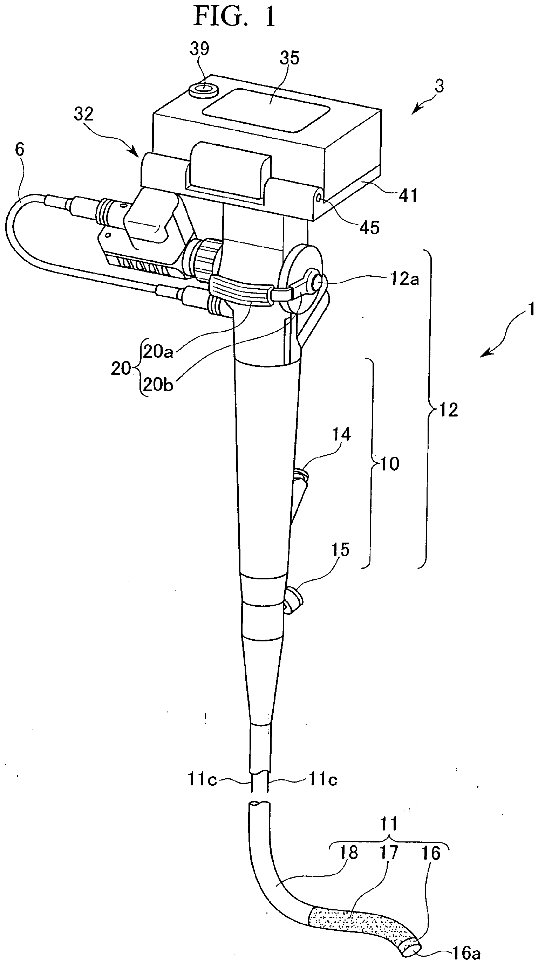

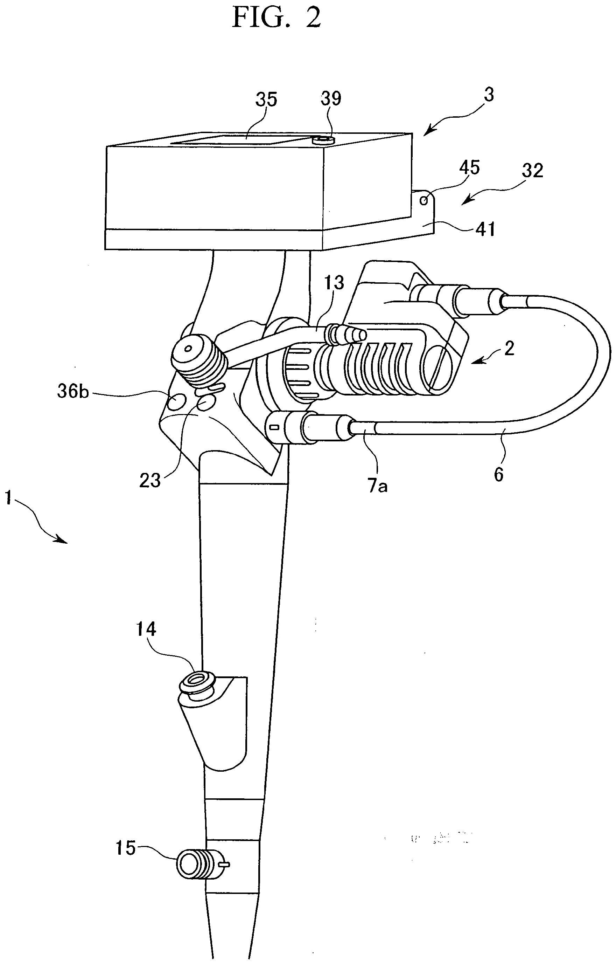

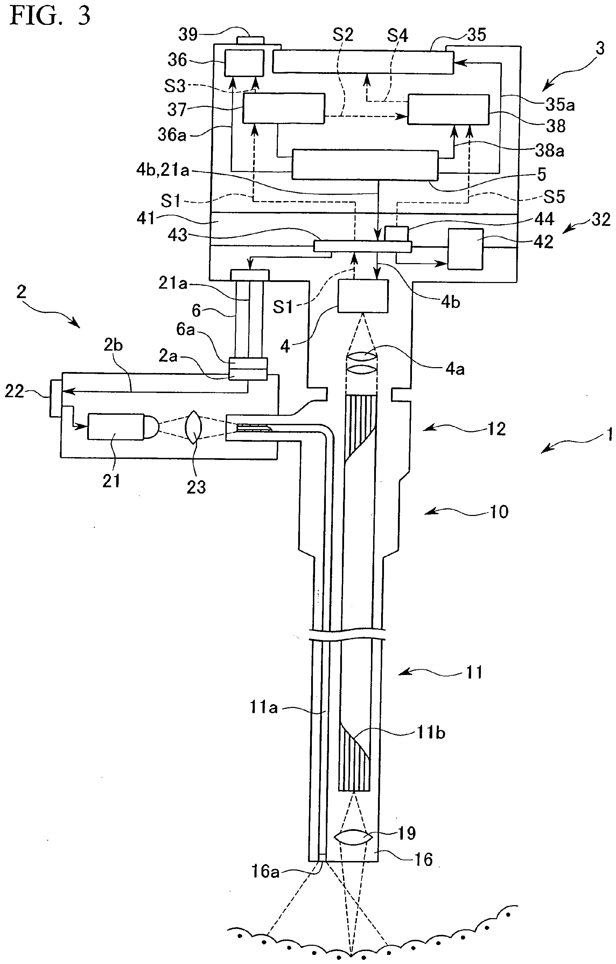

The first embodiment of the present invention illustrated in FIG. 1 through FIG. 7 will now be described.

As shown in FIG. 1 through FIG. 3, as principal component elements, the endoscope device of the present invention is provided with an endoscope 1, a light source apparatus 2 that generates illumination light for illuminating a subject, and a video image displaying device (i.e., a video image displaying device) 3 that converts an image of the subject that has been obtained by the endoscope 1 into a video image and displays it.

The endoscope 1 is provided with an insertion section 11 whose distal end is inserted into an observation portion, and an operation section 12 that is used to bend the distal end of the insertion section 11. In addition, the endoscope 1 is also provided with an imaging element (i.e., imaging device) 4 such as a CCD or the like that receives light from an image (i.e., from light) that is guided by an image guide 11b (described below), and a condenser lens 4a that forms an image on the light receiving portion of the imaging device 4. The insertion section 11 is formed in an elongated configuration having flexibility and is connected to one end of the operating section 12. The insertion section 11 is provided with a hard distal end portion 16 that is located at a distal end thereof, a bending portion 17 that is provided so as to continue from the distal end portion 16, and a flexible portion 18 that is provided so as to continue from the bending portion 17 and is connected to the operating section 12. The distal end portion 16 is provided with an objective lens 19 that forms an image from reflected light that is reflected from the subject which has been illuminated by the illumination light, and an illumination window 16a through which the illumination light is irradiated. A light guide 11a that guides illumination light from the light source apparatus 2 to the distal end portion 16, and an image guide 11b that guides images formed on the objective lens 19 to the imaging element 4 are incorporated in the bending portion 17 and flexible portion 18. Note that, in some cases, the imaging element 4 may be provided in the distal end portion 16 (described below).

The operating section 12 is provided with a gripping portion 10 that an operator uses to grip the endoscope 1, and a bending operation lever 20 that is used to bend the bending portion 17 in a desired direction via two wires 11c that pass through the insertion portion 11. The gripping portion 10 is formed in a rod shape such that it is able to be gripped by wrapping the thumb and the other fingers around it. The gripping portion 10 is provided with a suction cap 13 that is used for suctioning liquids such as body fluids and the like, a forceps insertion aperture 14 that is used to insert treatment tools such as forceps and the like, and an aeration cap 15 that is used to feed air into the interior of the endoscope 1 during inspections to check for leakages in the endoscope 1. A suction apparatus is connected by a tube (not shown) to the suction cap 13 and body fluids and the like can be suctioned through the suction cap 13 by operating the suction apparatus. An air supply apparatus can be connected by a tube (not shown) to the aeration cap 15 and, by operating the air supply apparatus, air can be supplied through the aeration cap 15 to the endoscope 1 so that a leakage inspection of the interior of the endoscope 1 can be performed. An image recording switch 36b that causes video images that are displayed on the video image displaying device 3 to be recorded on an image recording device 36 (described below) is provided in the operation section 12.

The bending operation lever 20 is provided adjacent to the gripping portion 10 such that it can be operated by the thumb of the hand gripping the gripping section 10. The bending operation lever 20 is formed in an L shape that is made up of a distal end portion 20a that is operated by the thick part of the thumb holding the gripping portion 10, and a base end portion 20b that is connected to one end of the distal end portion 20a, and is axially supported at the base end portion 20b on a shaft 12a, which is provided on the operating section 12, such that it can swing vertically. In the bending operation lever 20, the bending portion 17 can be made to bend freely by pushing or pulling the distal end portion 20a thereof using the thumb so that tensile force is applied to one of the wires 11c while thrust force is applied to the other of the wires 11c.

The light source apparatus 2 is provided with a light source lamp 21, a finger switch 22 that an operator uses to turn on or turn off the light source lamp 21 as desired, and a condenser lens 23 that condenses illumination light generated by the light source lamp 21. A connector 2a to which a power supply cable 6 (described below) can be removably connected is also provided in the light source apparatus 2. The light source lamp 21, the finger switch 22, and the connector 2a are connected in series by a power supply line 2b that is built into the power supply apparatus 2. Illumination light that is generated by the light source lamp 21 is condensed by the condenser lens 23, guided by the light guide 11a, and then emitted through the illumination window 16a so as to illuminate the interior of a body cavity.

The video image displaying device 3 is supported via a supporting portion 32 (i.e., a first supporting portion) at another end of the operation section 12. The video image displaying device 3 is provided with a display element 35 such as an LCD that converts observed images of a subject into video images and then displays them, an image recording device 36 that records images of the subject, an imaging element control circuit 37 that converts images of the subject that have been imaged by the imaging element 4 into signals and then outputs them, and a display element control circuit (i.e., a video image display control device) 38 that converts signals output from the imaging element control circuit 37 into images and then displays them on the display element 35. In addition, a startup switch 39 that turns the endoscope device ON and OFF is provided in the video image displaying device 3.

A battery 5 that supplies power respectively to the light source apparatus 2, the imaging element, and the video image displaying device 3 is replaceably fitted inside the video image displaying device 3. The battery 5 is a secondary battery that can be used by being repeatedly recharged.

The supporting portion 32 supports the video image displaying device 3 such that it can pivot around the longitudinal axis of the operation section 12. The supporting portion 32 is provided with a mounting base that supports the video image displaying device 3, a motor (i.e., a pivot drive device) 42 that causes the video image displaying device 3 to pivot around an axial line of the endoscope 1, and a slip ring 43 that is provided between the mounting base 41 and the motor 42 and that maintains an electrical connection between the video image displaying device 3, the imaging element 4, and the light source apparatus 2, and an encoder (i.e., a direction and angle detecting device) 44 that detects a pivot direction and pivot angle of the video image displaying device 3.

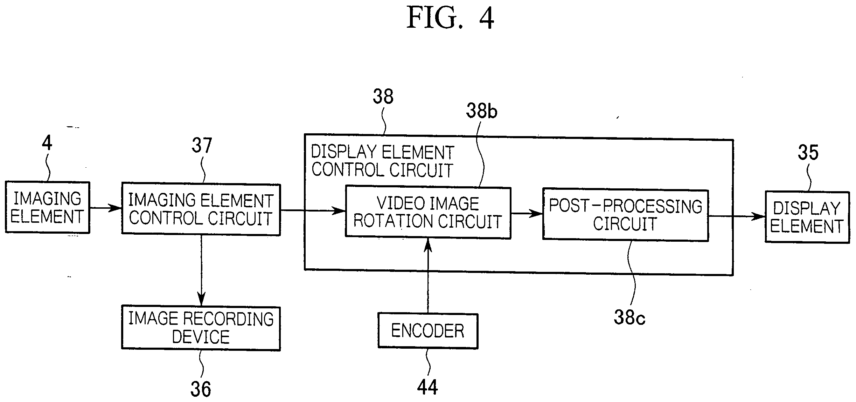

As shown in FIG. 4, the display element control circuit 38 is provided with a video image rotation circuit (i.e., a video image rotating device) 38b that causes video images acquired by the imaging element 4 to be rotated based on a pivot direction and pivot angle of the video image displaying device 3 that is detected by the encoder 44, and a post-processing circuit 38c that converts video images into video image signals that match the display element 35.

The mounting base 41 is supported by the slip ring 43 so as to be able to pivot around the longitudinal axis of the operation section 12. The video image displaying device 3 is supported via a hinge portion (i.e., a second supporting portion) 45 at one end of the mounting base 41. The video image displaying device 3 is supported so as to be able to pivot around an axis that is perpendicular to the longitudinal axis of the operation section 12, so that it is possible to change the orientation of the screen of the video image displaying device 3. A flexible print substrate is incorporated inside the hinge portion 45 so that, even if the orientation of the screen of the video image displaying device 3 is changed, an electrical connection is maintained between the video image displaying device 3 and the slip ring 43.

The motor 42 is provided inside the gripping portion 10. When the motor 42 revolves, the mounting base 41 that is supported on the slip ring 43 pivots around the longitudinal axis of the operation section 12.

The slip ring 43 maintains an electrical connection between the battery 5, the imaging element 4, and the light source lamp 21 such that, even if the video image displaying device 3 is pivoted around the longitudinal axis of the operation section 12, it is still possible to supply power to the imaging element 4 and the light source lamp 21. The slip ring 43 also maintains an electrical connection between the imaging element 4 and the imaging element control circuit 37 so that, even if the video image displaying device 3 is pivoted around the longitudinal axis of the operation section 12, it is still possible to transmit video image signals acquired by the imaging element 4 to the imaging element control circuit 37. The encoder 44 detects the pivot direction and pivot angle when the video image displaying device 3 is pivoted around the longitudinal axis of the operation section 12, and transmits this pivot direction and pivot angle to the display element control circuit 38.

The light source apparatus 2 is connected by the power supply cable 6 that encloses a power supply line 21a (described below) to the endoscope 1. The power supply cable 6 is fixed to the endoscope 1 side, while a distal end thereof is provided with a connector 6a. The connector 6a is removably connected to the connector 2a of the light source apparatus 2.

A power supply line 4b that supplies power from the battery 5 to the imaging element 4 is provided via the slip ring 43 between the battery 5 and the imaging element 4. A power supply line 21 a that supplies power from the battery 5 to the light source apparatus 2 is provided via the slip ring 43 between the battery 5 and the connector 6a.

A power supply line 35a that supplies power to the display element 4 is provided between the display element 35 and the battery 5, while a power supply line 36a that supplies power to the image recording device 36 is provided between the image recording device 36 and the battery 5. In addition, a power supply line 37a that supplies power to the imaging element control circuit 37 is provided between the imaging element control circuit 37 and the battery 5, while a power supply line 38a that supplies power to the display element control circuit 38 is provided between the display element control circuit 38 and the battery 5.

A signal line S1 that transmits video image signals acquired by the imaging element 4 to the imaging element control circuit 37 is provided via the slip ring 43 between the imaging element 4 and the imaging element control circuit 37. A signal line S2 that transmits video image signals that have been input into the imaging element control circuit 37 to the display element control circuit 38 is provided between the imaging element control circuit 37 and the display element control circuit 38, while a signal line S3 that transmits video image signals that have been input into the imaging element control circuit 37 to the image recording device 36 is provided between the imaging element control circuit 37 and the image recording device 36. A signal line S4 that inputs video image signals that have been input into the display element control circuit 38 to the display element 31 is provided between the display element control circuit 38 and the display element 35, while a signal line S5 that inputs a pivot direction and pivot angle of the video image displaying device 3 that have been detected by the encoder 44 into the display element control circuit 38 is provided between the slip ring 43 and the display element control circuit 38.

In an endoscope device that is structured in the manner described above, the video image displaying device 3 is mounted on a top end of the endoscope 1 via the supporting portion 32. In addition, it is possible for the video image displaying device 3 to be pivoted around the longitudinal axis of the operation section 12 using the supporting portion 32. Furthermore, it is also possible for the video image displaying device 3 to be pivoted around an axis that is perpendicular to the longitudinal axis of the operation section 12. As a result, if an operator is unable to obtain a good view of an image of a subject that is displayed on the screen of the video image displaying device 3 while operating the endoscope device, then the operator or an assistant is able to arbitrarily alter the orientation and angle of the screen of the video image displaying device such that they are able to obtain a good view of an image of a subject that is displayed on the screen.

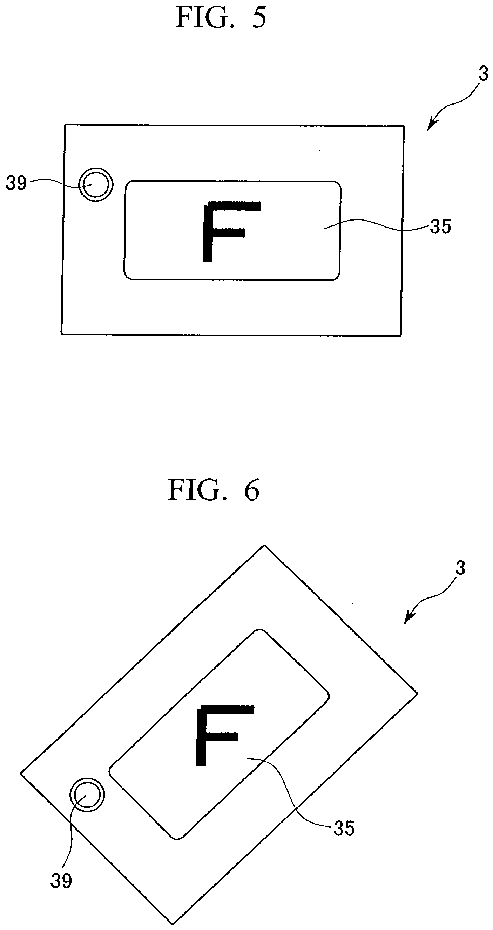

When an operator pivots the video image displaying device 3 so as to alter the angle, the screen itself rotates around an axis that is substantially orthogonal to the screen. Therefore, a video image displayed on the screen also rotates together with the screen. For example, as shown in FIG. 5, a video image (i.e., the alphabet letter F) displayed on the screen becomes obliquely tilted. In cases such as this, the display element control circuit 38 rotates the image of the subject that is displayed on the video image displaying device 3 in the opposite direction to the direction in which the video image display unit 3 was pivoted and by the same angle amount as the video image display unit 3 was pivoted. As a result, even if the screen itself is tilted, the way in which a video image appears as seen by the operator is the same as it was before the angle of the video image displaying device was altered.

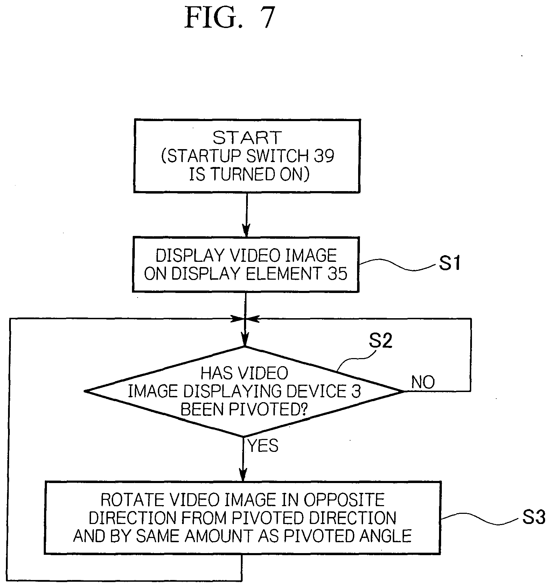

The processing shown in FIG. 7 is performed by the display element control circuit 38. When the startup switch 39 is pressed so that power is supplied from the battery 5 to the respective sections, the display element control circuit 38 displays an image of a subject on the display element 35 (step S1). Next, the display element control circuit 38 determines whether or not the video image displaying device 3 has pivoted around the operation section 12 (step S2). If the video image displaying device 3 has pivoted, the encoder 44 detects the direction in which the video image displaying device 3 has pivoted and the angle by which it has pivoted. At this point, the display element control circuit 38 rotates the image of the subject that is displayed on the video image displaying device 3 in the opposite direction from the pivot direction detected by the encoder 44 and by the same amount as the pivot angle that was detected by the encoder 44 (step S3). For example, as shown in FIG. 6, even if the screen itself is tilted, the video image displayed on the screen remains upright and is not tilted. If the video image displaying device 3 has not been pivoted, the display element control circuit 38 repeats the processing of step S2. The display element control circuit 38 continuously executes the above described processing until the startup switch 39 is again pressed and the power supply from the battery 5 to the respective sections is cut off.

In the endoscope device of the present embodiment, because processing such as that described above is performed, difficulties do not arise as an operation or examination performed by an operator or assistant progresses.

In the endoscope device of the present embodiment, the relative positions of the operation section 12 and the video image displaying device 3 are maintained by the supporting portion 32 and the hinge portion 45 as long as there is no intentional external application of pressure. Namely, even if force that has not been intentionally applied to the video image displaying device by an operator, for example, gravity or inertial force that is generated when the endoscope device is abruptly moved is applied thereto, the relative positions of the operation section 12 and the video image displaying device 3 are kept unchanged. Consequently, the endoscope device is easy to handle.

Note that in the present embodiment the video image displaying device 3 is supported such that it can pivot around the longitudinal axis of the operation section 12, however, it may also be supported such that it can pivot around an axis that is substantially parallel with the longitudinal axis of the operation section 12. Moreover, it is also possible to use a semiconductor sensor such as a biaxial sensor or triaxial sensor instead of the encoder 44. These exhibit high performance qualities and are also able to be obtained extremely cheaply and, accordingly, have considerable merits for commercialization of the endoscope device. Note that, in the present invention, because it is sufficient if detection is possible in a single axial direction, if a biaxial sensor or triaxial sensor is used, then only one channel thereof is used.

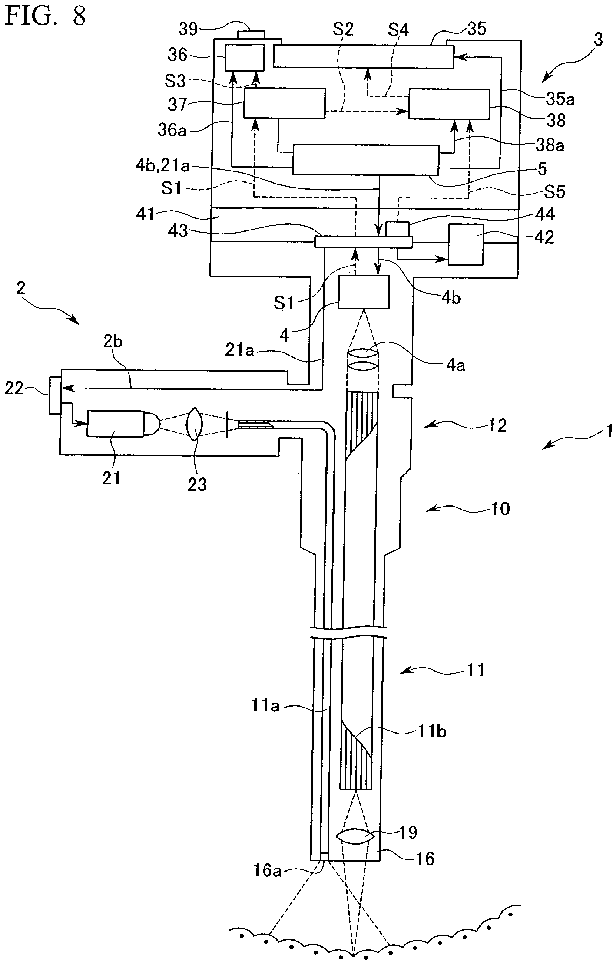

In the present embodiment, the endoscope 1 and the light source apparatus 2 are connected by the power supply cable 6, however, the endoscope device of the present invention is not limited to the above described structure and, for example, as shown in FIG. 8, it is also possible to employ a structure in which the endoscope 1 and the light source apparatus 2 are formed integrally.

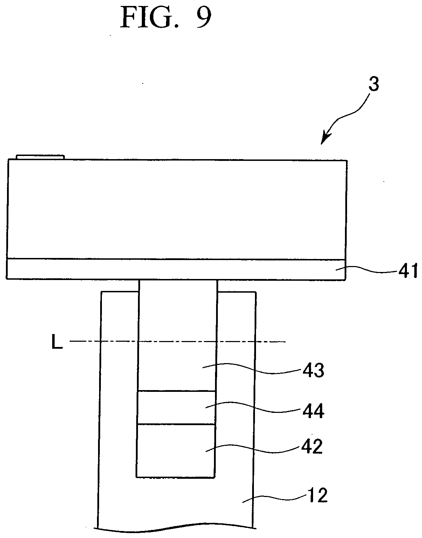

As shown in FIG. 9, in the present embodiment, it is also possible for the video image displaying device 3 to be supported so as to be able to pivot around an axis L that is orthogonal to the longitudinal axis of the operation section 12, and for the slip ring 43 and the encoder 44 to be provided on the axis L and for the motor 42 to also be provided that drives the video image displaying device 3 around the axis L. In this example, if the video image displaying device 3 is pivoted around the operation section 12, the pivot direction and pivot angle are detected by the encoder 44 and the motor 42 then causes the video image displaying device 3 to pivot in the opposite direction from the pivot direction detected by the encoder 44 and by the same amount as the detected angle.

### (Second embodiment)

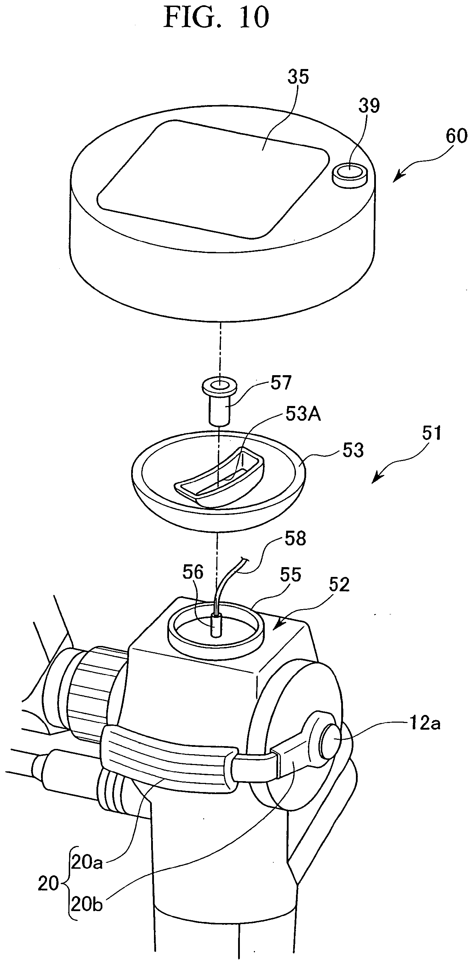

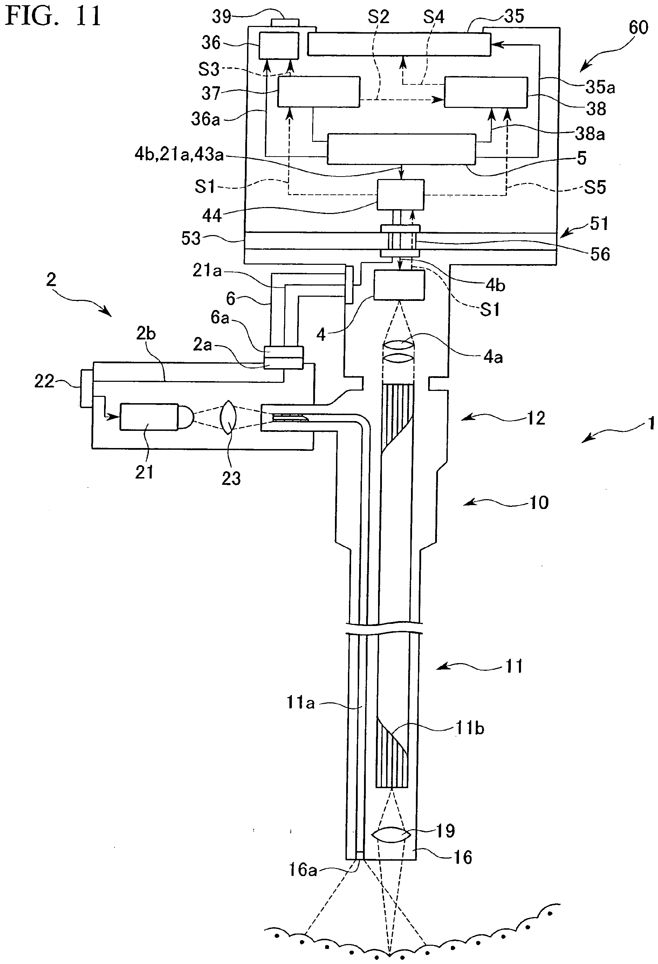

The second embodiment of the present invention illustrated in FIG. 10 and FIG. 11 will now be described. Note that component elements that have already been described in the first embodiment are given the same symbols and a description thereof is omitted.

In the above described first embodiment, the video image displaying device 3 is supported by the supporting portion 32 provided on the mounting base 41 such that it can pivot around the longitudinal axis of the operation section 12. In contrast to this, in the second embodiment, a video image displaying device 60 is supported by a supporting portion 51 such that it can pivot around the longitudinal axis of the operation section 12 and is also supported such that it can slide around an axis that is perpendicular to this longitudinal axis.

The supporting portion 51 is equipped with a base portion 52 that is provided at a top end of the operation section 12, and with a pivot slide mechanism 53 that is able to pivot around the longitudinal axis of the operation section 12 and is also able to slide around an axis that is perpendicular to this longitudinal axis.

The base portion 52 is formed by a cylindrical convex portion 55 that is formed on a top end of the operation section 12, and a cylindrical pivot shaft 56 that is provided in a center of the convex portion 55.

A male thread that engages with a female thread that is formed on an inner circumferential surface of a cylindrical screw 57 is formed on a circumferential surface of the pivot shaft 56. A cable 58 that encloses the power supply lines 4b and 21a (described below) and the signal line S1 passes through the center of the pivot shaft 56.

The pivot slide mechanism 53 is a bowl-shaped component and a through hole in the form of an elongated hole 53 is formed in the center thereof. The pivot shaft 56 is fitted into this elongated hole 53A, and the cylindrical screw 57 is screwed onto the pivot shaft 56 that protrudes from the elongated hole 53A and the cylindrical screw 57 is fixed to the base portion 52. As a result, the pivot slide mechanism 53 is supported by the convex portion 55 of the base portion 52 and the cylindrical screw 57. The pivot slide mechanism 53 is able to pivot around the cylindrical screw 57 and is also able to slide along the elongated hole 53A that penetrates the cylindrical screw 57. The video image displaying device 60 is fixed to a top surface of the pivot slide mechanism 53. As a result, the video image displaying device 60 is able to pivot around the longitudinal axis of the operation section 12 and is also able to slide around an axis that is perpendicular to this longitudinal axis. The encoder 44 detects the pivot direction and pivot angle of the rotation slide mechanism 53 relative to the cylindrical screw 57.

In an endoscope device that is structured in the manner described above, in the same way as in the endoscope device in the above described first embodiment, it is also possible for an operator or for an assistant to obtain a consistently good view irrespective of the viewing angle of the liquid crystal display screen used in the display element 35.

In the present embodiment, the video image displaying device 60 is supported by the supporting portion 51 such that it can pivot around the longitudinal axis of the operation section 12, however, it is also possible for the video image displaying device 60 to be supported so as to be able to pivot around an axis that is substantially parallel to the longitudinal axis of the operation section 12. Moreover, in the same way as in the above described first embodiment, it is also possible to employ a structure in which a motor is provided inside the video image displaying device 60 and the video image displaying device 60 is made to pivot by this motor around the longitudinal axis of the operation section 12 or around an axis that is substantially parallel to this longitudinal axis.

### (Third embodiment)

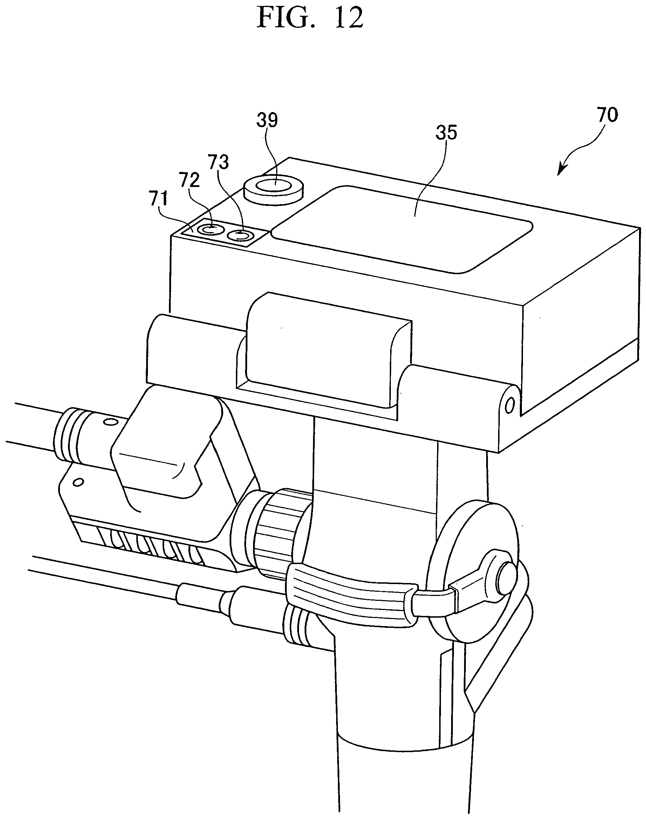

The third embodiment of the present invention illustrated in FIG. 12 through FIG. 14 will now be described. Note that component elements that have already been described in the first embodiment are given the same symbols and a description thereof is omitted.

In the above described first embodiment, the image of the subject that is displayed on the video image displaying device 3 is rotated by the same amount as the pivot angle detected by the encoder 44 in the opposite direction to the pivot direction of the video image displaying device 3 that was detected by the encoder 44. In contrast, in the third embodiment, an operator or another person such as an assistant is able to rotate an image of a subject that is displayed on a video image displaying device 70 to a desired direction and by a desired angle.

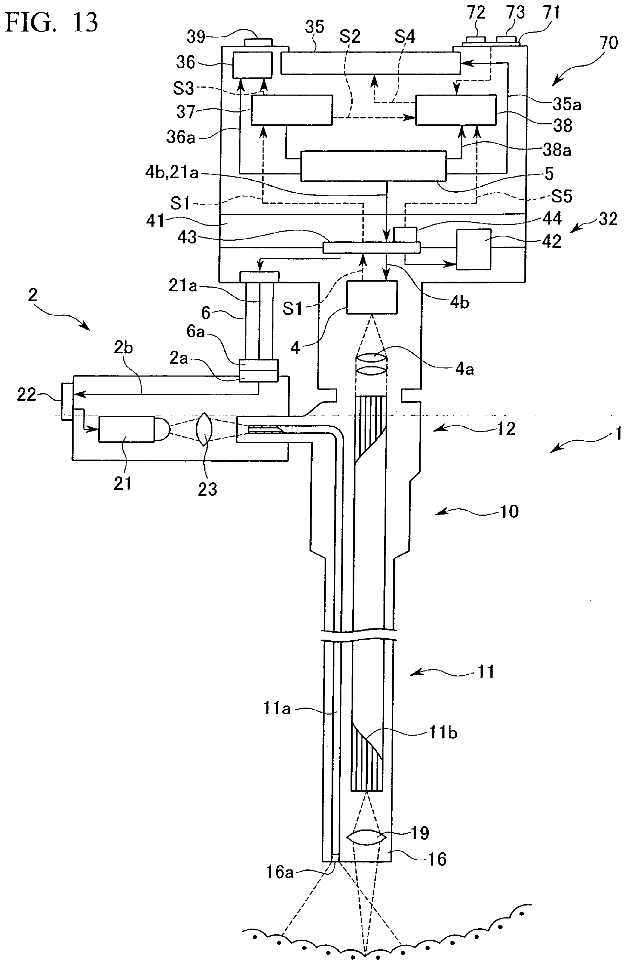

As shown in FIG. 12 and FIG. 13, a rotation operation panel 71 is provided on a top surface of the video image displaying device 70. A right rotation button (i.e., rotation operation portion) 72 for rotating an image of a subject that is displayed on the video image displaying device 70 in a rightward direction and a left rotation button (i.e., rotation operation portion) 73 for rotating an image of a subject in a leftward direction are provided in the rotation operation panel 71.

If an operator or another person such as an assistant presses the right rotation button 72, the image of the subject that is displayed on the video image displaying device 3 is rotated in a rightward direction, while if the left rotation button 73 is pressed, the image of the subject that is displayed on the video image displaying device 3 is rotated in a leftward direction. As long as the right rotation button 72 is being pressed, the image of the subject continues to rotate in a rightward direction, while as long as the left rotation button 73 is pressed, the image of the subject continues to rotate in a leftward direction. If the button is released, the rotation of the image is stopped.

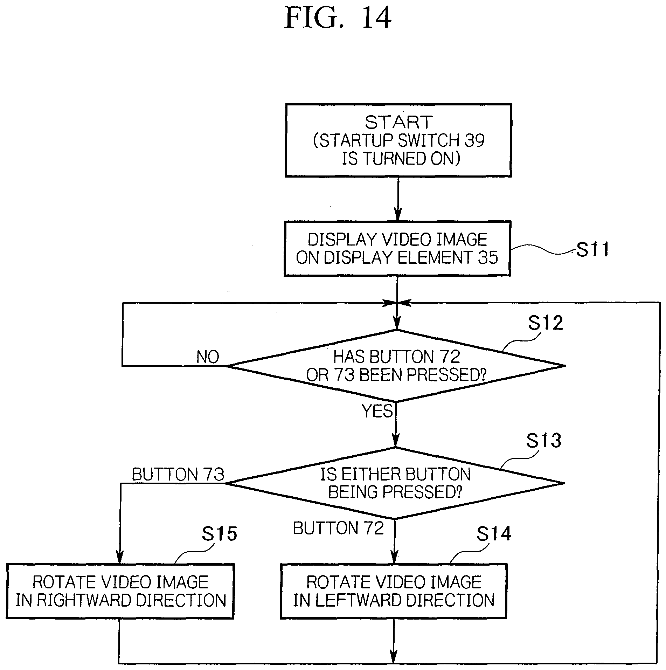

The processing shown in FIG 14 is performed in the display element control circuit 38. When the startup switch 39 is pressed and power is supplied from the battery 5 to the respective portions, the display element control circuit 38 displays an image of the subject on the display element 35 (step S11). Next, the display element control circuit 38 determines whether or not the right rotation button 72 or the left rotation button 73 is being pressed (step S12). If either one of these buttons is being pressed, the display element control circuit 38 determines which one of the right rotation button 72 and the left rotation button 73 is being pressed (step S 13). If the right rotation button 72 is being pressed, the display element control circuit 38 rotates the image of the subject that is displayed on the video image displaying device 3 in a rightward direction (step S14). If the left rotation button 73 is being pressed, the display element control circuit 38 rotates the image of the subject that is displayed on the video image displaying device 3 in a leftward direction (step S 15). The display element control circuit 38 continuously executes the above described processing until the startup switch 39 is again pressed and the power supply from the battery 5 to the respective portions is cut off.

According to an endoscope device that is structured in the manner described above, an operator or another person such as an assistant is able to optionally rotate an image of a subject that is displayed on the video image displaying device 70 so that they can easily view it.

Note that in the present embodiment, two buttons, namely, the right rotation button 72 and the left rotation button 73 are provided and an image can be rotated in both the rightward and leftward directions by operating these buttons, however, it is also possible for only the right rotation button 72 to be provided and for the image to be rotated only in the rightward direction by operating this button, or for only the left rotation button 72 to be provided and for the image to be rotated only in the leftward direction by operating this button.

### (Fourth embodiment)

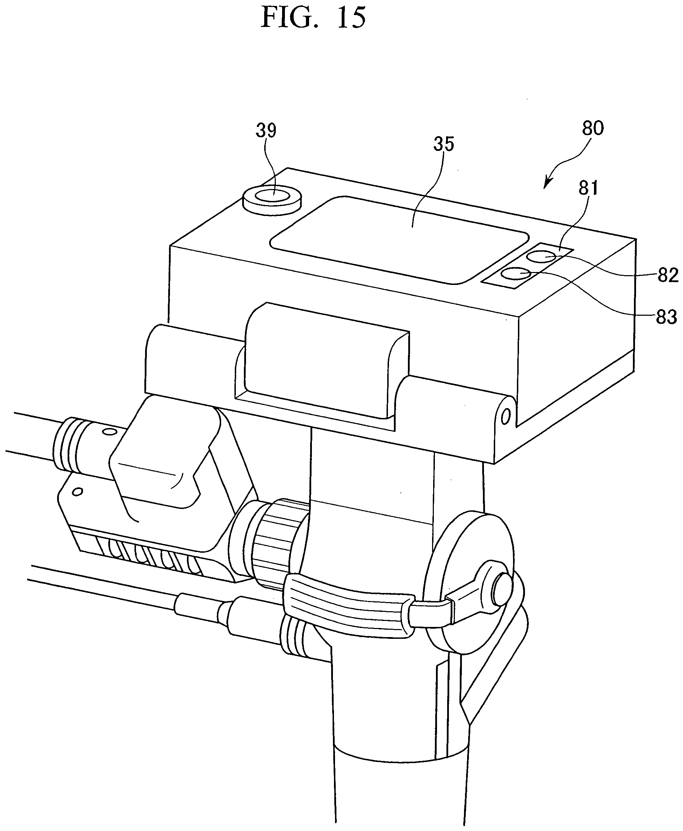

The fourth embodiment of the present invention illustrated in FIG. 15 through FIG. 17 will now be described. Note that component elements that have already been described in the first embodiment are given the same symbols and a description thereof is omitted.

As shown in FIG. 15, an image recording selection panel 81 is provided on a top surface of a video image displaying device 80 of the endoscope device of the present embodiment. Two selection buttons 82 and 83 for selecting a recording state for recording an image on the image recording device 36 are provided on the image recording selection panel 81.

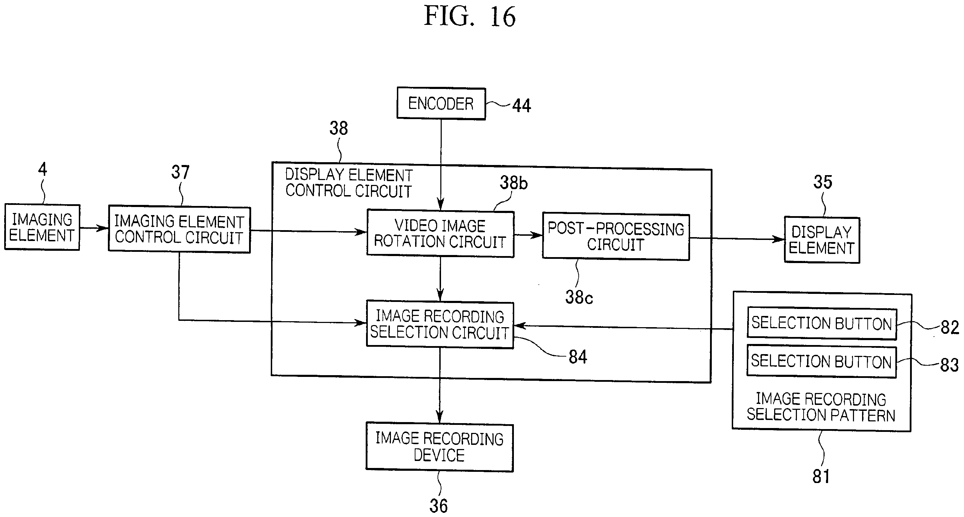

As shown in FIG. 16, the display element control circuit 38 is provided with an image recording selection circuit (i.e., an image recording selection device) 84. The image recording selection device 84 selects whether to record on the image recording device 36 an image of a subject that is displayed on the display element 35 in the video image displaying device 80 in a state prior to the video image rotation circuit 38b being operated or in a state after the video image rotation circuit 38b has been operated.

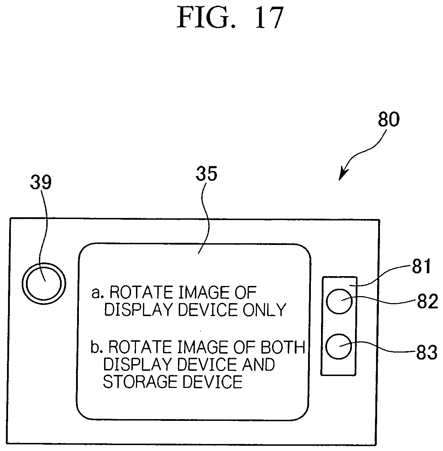

If the startup switch 39 is pressed and the endoscope device is started up, as shown in FIG. 17, an initial setting screen that asks for a selection of the recording state for recording an image onto the image recording device 36 is displayed on the screen of the video image displaying device 80. Two modes are displayed on this initial setting screen, namely, [a. rotate image only on display device] and [b. rotate image on display device and recording device]. The (a.) mode corresponds to the selection button 82 while the (b.) mode corresponds to the selection button 83. If the selection button 82 is pressed, the (a.) mode is selected, while if the selection button 83 is pressed, the (b.) mode is selected.

If the selection button 82 is pressed so that the (a.) mode is selected, then if the video image displaying device 3 is pivoted, the image of the subject is pivoted in the opposite direction and displayed on the video image displaying device 80. At this time, even if the image recording device 36 is operated, the recorded image is recorded in a state of not having been pivoted in the opposite direction. If, on the other hand, the selection button 83 is pressed so that the (b.) mode is selected, then if the video image displaying device 3 is pivoted, the image of the subject is pivoted in the opposite direction and displayed on the video image displaying device 80. At this time, if the image recording device 36 is operated, the recorded image is recorded in a state of having been pivoted in the opposite direction.

In the endoscope device of the present invention, an operator or another person such as an assistant is able to arbitrarily select whether to record an image that is to be recorded on the image recording device 36 in a state prior to the video image rotation circuit 38b being operated, or in a state after the video image rotation circuit 38b has already been operated.

Note that in the present embodiment the selection buttons 82 and 83 are provided separately from the display element 35 in the video image displaying device 80, however, it is also possible for the display element 35 to be in the form of a touch panel and for the selection buttons to be provided on the screen thereof. Moreover, it is also possible to not make the initial settings and to make it possible to switch a setting by operating the selection buttons 82 and 83 while the endoscope device is being operated.

The present invention is not limited to the above described embodiments and various modifications may be made thereto insofar as they do not depart from the spirit or scope of the present invention.

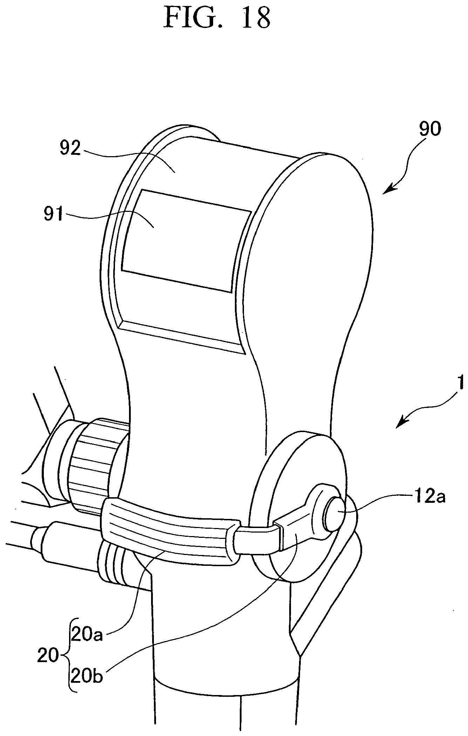

For example, as shown in FIG. 18, it is also possible for a video image displaying device 90 to be able to pivot around an axis that is perpendicular to the longitudinal axis of the operation section 12. The video image displaying device 90 is formed integrally with the endoscope 1 and is provided with a film-shaped display element 91 that has flexibility, a circular column-shaped roulette (KNURL ??) 92 to whose circumferential surface the display element 91 is attached, and a motor (not shown) that is provided inside the video image displaying device 90 and that causes the roulette 92 to pivot in a circumferential direction. The display element 35, the image recording device 36, the imaging element control circuit 37, the display element control circuit 38 and the battery 5 are placed inside the operation section 12.

In this video image displaying device 90, if the motor is operated, the display element 91 is moved in the circumferential direction of the roulette 92. As a result, an image of the subject that is displayed on the image element 91 is moved in the circumferential direction of the roulette 92.

In an endoscope device that has this type of structure as well, in the same way as is described above, it is possible for an operator or for an assistant to obtain a consistently good view of a subject irrespective of the viewing angle of the liquid crystal display screen used in the display element 91.

Note that it is also possible to provide the display element 91 such that it covers the circumferential surface of the roulette 92 and for an image of a subject to be displayed only on a portion of the display element 91. By driving the motor, the position of the display of the display element 91 is moved in the circumferential direction of the roulette 92.

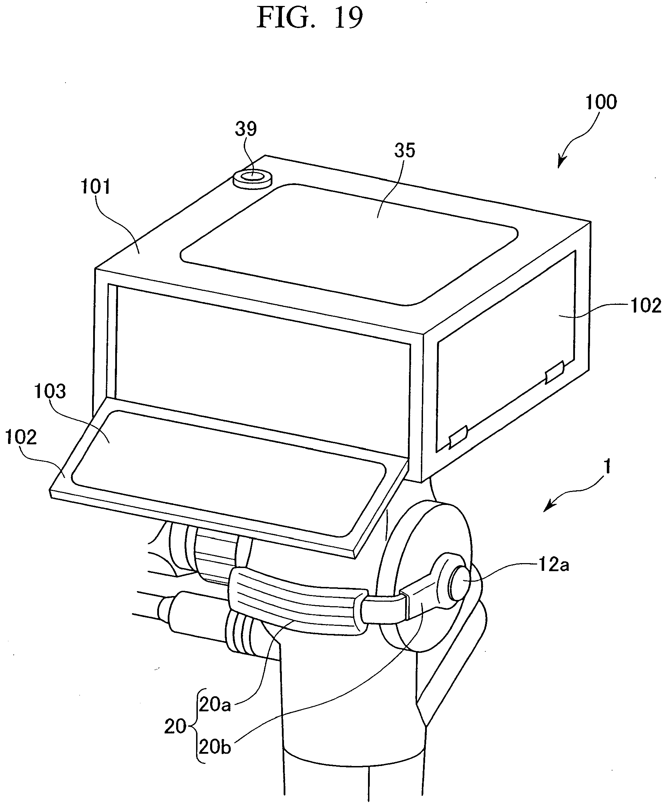

Moreover, as shown in FIG. 19, it is also possible to use a video image displaying device 100 in which a plurality of display elements 103 are provided on circumferential surfaces of a housing 101 of the video image displaying device 100. The video image displaying device 100 is placed on the top end of the operation section 12 and the display element 35, the image recording device 36, the imaging element control circuit 37, the display element control circuit 38, and the battery 5 are provided inside the substantially rectangular parallelepiped-shaped housing 101. Lid bodies 102 that can be opened and closed freely via a hinge mechanism (not shown) are provided on each of the side surfaces of the housing 101. A display element 103 is provided on each side surface of the housing 101 that is opened and closed by a lid body 102. The display elements 103 display an image of a subject in the same way as the display element 35.

In the endoscope device that is structured in the manner described above, the video image displaying device 100 is not supported such that it can pivot around an axis that is substantially parallel to or that matches the longitudinal axis of the operation section 12. However, an operator or assistant is able to open a lid body 102 if necessary and observe an image of a subject that is displayed on the display element 103 thereof. Accordingly, it is possible for an operator or for an assistant to obtain a consistently good view of a subject irrespective of the viewing angle of the liquid crystal display screen used in the display element 35 and the display elements 103.

Note that it is also possible for the display elements 103 that are provided in the side surfaces to receive a supply of power from the battery 5 and display an image of a subject only when the respective lid body 102 has been opened. By employing this type of structure, it is possible to avoid power being consumed by the display elements 83.

While preferred embodiments of the invention have been described and illustrated above, it should be understood that these are exemplary of the invention and are not to be considered as limiting. Accordingly, the invention is not to be considered as limited by the foregoing description and is only limited by the scope of the appended claims.

### INDUSTRIAL APPLICABILITY

The endoscope device of the present invention can be favorably used not only in the above described medical applications but also in industrial applications.

## Claims

1. An endoscope device comprising:
an imaging device (4) configured to image an observation image of a subject;
an endoscope (1) having a grippable operation section (12);
a video image displaying device (3) configured to convert an image signal from the imaging device (4) into a video image and display the video image; and
a first supporting section (32) that is provided in the operation section (12) and is configured to support the video image displaying device (3) so that the video image displaying device (3) is pivotable around a longitudinal axis of the operation section (12) or about an axis substantially parallel to the longitudinal axis,
**characterized by**
a video image rotating device (38b) configured to rotate the image of the subject displayed on the video image displaying device (3) about an axis substantially orthogonal to the screen of the video image displaying device (3); and
a direction and angle detecting device (44) configured to, when the video image displaying device (3) is pivoted relative to the operation section (12), detect a direction in which the video image displaying device (3) has been pivoted and an angle by which the video image displaying device (3) has been pivoted, wherein
the video image rotating device (38b) is configured to rotate the image of the subject that is displayed on the video image displaying device (3) in an opposite direction from the pivot direction detected by the direction and angle detecting device (44) and by the same amount as the pivot angle detected by the direction and angle detecting device (44).

2. The endoscope device according to claim 1, further comprising:
a second supporting section (45) configured to support the video image displaying device (3) so that the video image displaying device (3) is pivotable around an axis that is perpendicular to the longitudinal axis or to an axis that is substantially parallel with the longitudinal axis.

3. The endoscope device according to any one of claims 1 and 2, wherein
the first supporting section (32) is able to arbitrarily fix the position of the video image displaying device (3) relative to the operation section (12) and maintain the relative positions of the two as long as there is no intentional external application of pressure.

4. The endoscope device according to any one of claims 1 to 3, wherein
the second supporting section (45) is able to arbitrarily fix the position of the video image displaying device (3) relative to the operation section (12) and maintain the relative positions of the two as long as there is no intentional external application of pressure.

5. The endoscope device according to any one of claims 1 to 4, further comprising a pivot drive device (42) configured to drive the video image displaying device (3) in a pivot direction around the longitudinal axis or around an axis that substantially parallel with the longitudinal axis.

6. The endoscope device according to claim 1, further comprising a rotation operation section (72, 73) configured to be operated by an operator or by another person, wherein
the video image rotating device (38b) is configured to rotate an image of the subject displayed on the video image displaying device (3) in a predetermined direction in accordance with an operation of the rotation operation section (72, 73).

7. The endoscope device according to claim 1, further comprising:
an image recording device (36) configured to record an image of the subject displayed on the video image displaying device (3); and
an image recording selection device (84) configured to select whether to record on the image recording device (36) an image of the subject that is displayed on the video image displaying device (3) in a state prior to the video image rotating device (38b) being operated or in a state after the video image rotating device (38b) has been operated.

## Patentansprüche

1. Endoskopeinrichtung, die umfasst:
eine Bildgebungseinrichtung (4), die dazu eingerichtet ist, ein Beobachtungsbild eines Subjekts abzubilden;
ein Endoskop (1), das einen greifbaren Betätigungsabschnitt (12) hat;
eine Videobild-Anzeigeeinrichtung (3), die dazu eingerichtet ist, ein Bildsignal von der Bildgebungseinrichtung (4) in ein Videobild umzuwandeln und das Videobild anzuzeigen; und
einen ersten Stützabschnitt (32), der in dem Betätigungsabschnitt (12) vorgesehen und dazu eingerichtet ist, die Videobild-Anzeigeeinrichtung (3) so abzustützen, dass die Videobild-Anzeigeeinrichtung (3) um eine Längsachse des Betätigungsabschnitts (12) oder um eine zu der Längsachse im Wesentlichen parallele Achse schwenkbar ist,
**gekennzeichnet durch**
eine Videobild-Rotationseinrichtung (38b), die dazu eingerichtet ist, das auf der Videobild-Anzeigeeinrichtung (3) angezeigte Bild des Subjekts um eine zu dem Bildschirm der Videobild-Anzeigeeinrichtung (3) im Wesentlichen orthogonale Achse zu drehen; und
eine Richtungs- und Winkelerfassungseinrichtung (44), die dazu eingerichtet ist, wenn die Videobild-Anzeigeeinrichtung (3) relativ zu dem Betätigungsabschnitt (12) geschwenkt wird, eine Richtung, in die die Videobild-Anzeigeeinrichtung (3) geschwenkt worden ist, und einen Winkel, um den die Videobild-Anzeigeeinrichtung (3) geschwenkt worden ist, zu erfassen, wobei
die Videobild-Rotationseinrichtung (38b) dazu eingerichtet ist, das Bild des Subjekts, das auf der Videobild-Anzeigeeinrichtung (3) angezeigt wird, in eine zu der von der Richtungs- und Winkelerfassungseinrichtung (44) erfasste Schwenkrichtung entgegengesetzte Richtung und um den gleichen Betrag wie den von der Richtungs- und Winkelerfassungseinrichtung (44) erfasste Schwenkwinkel zu drehen.

2. Endoskopeinrichtung nach Anspruch 1, die ferner umfasst:
einen zweiten Stützabschnitt (54), der dazu eingerichtet ist, die Videobild-Anzeigeeinrichtung (3) so abzustützen, dass die Videobild-Anzeigeeinrichtung (3) um eine Achse schwenkbar ist, die senkrecht zu der Längsachse oder eine zu der Längsachse im Wesentlichen parallele Achse ist.

3. Endoskopeinrichtung nach einem der Ansprüche 1 und 2, bei der
der erste Stützabschnitt (32) dazu in der Lage ist, die Position der Videobild-Anzeigeeinrichtung (3) relativ zu dem Betätigungsabschnitt (12) beliebig festzulegen und die relativen Positionen der beiden so lange beizubehalten, wie keine absichtliche Anwendung eines Drucks von außen erfolgt.

4. Endoskopeinrichtung nach einem der Ansprüche 1 und 3, wobei
der zweite Stützabschnitt (45) dazu in der Lage ist, die Position der Videobild-Anzeigeeinrichtung (3) relativ zu dem Betätigungsabschnitt (12) beliebig festzulegen und die relativen Positionen der beiden so lange beizubehalten, wie keine absichtliche Anwendung eines Drucks von außen erfolgt.

5. Endoskopeinrichtung nach einem der Ansprüche 1 und 4, die ferner eine Schwenkantriebseinrichtung (42) umfasst, die dazu eingerichtet ist, die Videobild-Anzeigeeinrichtung (3) in eine Schwenkrichtung um die Längsachse oder um eine Achse anzutreiben, die im Wesentlichen parallel zu der Längsachse ist.

6. Endoskopeinrichtung nach Anspruch 1, die ferner einen Rotationsbetätigungsabschnitt (72, 73) umfasst, der dazu eingerichtet ist, von einem Operateur oder von einer anderen Person betätigt zu werden, wobei
die Videobild-Rotationseinrichtung (38b) dazu eingerichtet ist, ein Bild des Subjekts, das auf der Videobild-Anzeigeeinrichtung (3) angezeigt wird, gemäß einer Betätigung des Rotationsbetätigungsabschnitts (72, 73) in eine vorbestimmte Richtung zu drehen.

7. Endoskopeinrichtung nach Anspruch 1, die ferner umfasst:
eine Bildaufzeichnungseinrichtung (38), die dazu eingerichtet ist, ein Bild des Subjekts, das auf der Videobild-Anzeigeeinrichtung (3) angezeigt wird, aufzuzeichnen; und
eine Bildaufzeichnungsauswahleinrichtung (84), die dazu eingerichtet ist, auszuwählen, ob auf der Bildaufzeichnungseinrichtung (38) ein Bild des Subjekts, das auf der Videobild-Anzeigeeinrichtung (3) angezeigt wird, in einem Zustand vor der Betätigung der Videobild-Rotationseinrichtung (38b) oder in einem Zustand nach der Betätigung der Videobild-Rotationseinrichtung (38b) aufgezeichnet werden soll.

## Revendications

1. Dispositif d'endoscope comprenant :
un dispositif (4) d'imagerie configuré pour imager une image d'observation d'un sujet ;
un endoscope (1) comportant une section d'actionnement (12) pouvant être saisie ;
un dispositif (3) d'affichage d'image vidéo configuré pour convertir un signal d'image provenant du dispositif (4) d'imagerie en une image vidéo et afficher l'image vidéo ; et
une première section de support (32) qui est prévue dans la section d'actionnement (12) et est configurée pour supporter le dispositif (3) d'affichage d'image vidéo de sorte que le dispositif (3) d'affichage d'image vidéo peut pivoter autour d'un axe longitudinal de la section d'actionnement (12) ou autour d'un axe sensiblement parallèle à l'axe longitudinal,
**caractérisé par**
un dispositif (38b) de rotation d'image vidéo configuré pour faire tourner l'image du sujet affichée sur le dispositif (3) d'affichage d'image vidéo autour d'un axe sensiblement orthogonal à l'écran du dispositif (3) d'affichage d'image vidéo ; et
un dispositif (44) de détection de sens et d'angle configuré pour, lorsque le dispositif (3) d'affichage d'image vidéo est pivoté par rapport à la section d'actionnement (12), détecter un sens dans lequel le dispositif (3) d'affichage d'image vidéo a été pivoté et un angle selon lequel le dispositif (3) d'affichage d'image vidéo a été pivoté, dans lequel
le dispositif (38b) de rotation d'image vidéo est configuré pour faire tourner l'image du sujet qui est affichée sur le dispositif (3) d'affichage d'image vidéo dans un sens opposé par rapport au sens de pivotement détecté par le dispositif (44) de détection de sens et d'angle et de la même quantité que l'angle de pivotement détecté par le dispositif (44) de détection de sens et d'angle.

2. Dispositif d'endoscope selon la revendication 1, comprenant en outre :
une deuxième section de support (45) configurée pour supporter le dispositif (3) d'affichage d'image vidéo de sorte que le dispositif (3) d'affichage d'image vidéo peut être pivoté autour d'un axe qui est perpendiculaire à l'axe longitudinal ou à un axe qui est sensiblement parallèle à l'axe longitudinal.

3. Dispositif d'endoscope selon l'une quelconque des revendications 1 et 2, dans lequel
la première section de support (32) est capable de fixer arbitrairement la position du dispositif (3) d'affichage d'image vidéo par rapport à la section d'actionnement (12) et de maintenir les positions relatives des deux tant qu'il n'y a aucune application de pression externe intentionnelle.

4. Dispositif d'endoscope selon l'une quelconque des revendications 1 à 3, dans lequel
la deuxième section de support (45) est capable de fixer arbitrairement la position du dispositif (3) d'affichage d'image vidéo par rapport à la section d'actionnement (12) et de maintenir les positions relatives des deux tant qu'il n'y a aucune application de pression externe intentionnelle.

5. Dispositif d'endoscope selon l'une quelconque des revendications 1 à 4, comprenant en outre un dispositif (42) d'entraînement en pivotement configuré pour entraîner le dispositif (3) d'affichage d'image vidéo dans un sens de pivotement autour de l'axe longitudinal ou autour d'un axe qui est sensiblement parallèle à l'axe longitudinal.

6. Dispositif d'endoscope selon la revendication 1, comprenant en outre une section (72, 73) d'actionnement en rotation configurée pour être actionnée par un opérateur ou par une autre personne, dans lequel
le dispositif (38b) de rotation d'image vidéo est configuré pour faire tourner une image du sujet affichée sur le dispositif (3) d'affichage d'image vidéo dans un sens prédéterminé conformément à un actionnement de la section (72, 73) d'actionnement en rotation.

7. Dispositif d'endoscope selon la revendication 1, comprenant en outre :
un dispositif (36) d'enregistrement d'image configuré pour enregistrer une image du sujet affichée sur le dispositif (3) d'affichage d'image vidéo ; et
un dispositif (84) de sélection d'enregistrement d'image configuré pour sélectionner l'enregistrement ou non sur le dispositif d'enregistrement d'image (36) d'une image du sujet qui est affichée sur le dispositif (3) d'affichage d'image vidéo dans un état avant que le dispositif (38b) de rotation d'image vidéo soit actionné ou dans un état après que le dispositif (38b) de rotation d'image vidéo a été actionné.
